# EUROPÄISCHE PATENTSCHRIFT

(11) **EP 0 743 306 B1**
(45) Veröffentlichungstag und Bekanntmachung des Hinweises auf die Patenterteilung: **17.01.2001**
(21) Anmeldenummer: 96107612.2
(22) Anmeldetag: 13.05.1996
(51) Int. Cl.: C07D 233/68, C07D 233/96

(54) **Verfahren zur Herstellung von gegebenenfalls 2-substituierten 5-Chlor-imidazol-4-carbaldehyden**
Process for the preparation of optionally 2-substituted 5-chloro-imidazole-4-carbaldehydes
Procédé pour la préparation des 5-chloro-imidazole-4-carbaldéhydes facultativement substitués sur la position 2

(30) Priorität: 17.05.1995 CH 145195
(43) Veröffentlichungstag der Anmeldung: 20.11.1996
(73) Patentinhaber: LONZA A.G., CH-3945 Gampel/Wallis (CH)
(72) Erfinder: Griffiths, Gareth, Dr., 3930 Visp (CH); Stucky, Gerhard, Dr., 3902 Brig-Glis (CH)
(74) Vertreter: Weinhold, Peter, Dr.rer.nat. Dipl.-Chem.

(56) Entgegenhaltungen:
- EP-A- 0 579 212
- EP-A- 0 614 890
- EP-A- 0 614 892
- EP-A- 0 653 422
- DE-A- 2 804 435
- US-A- 4 345 935
- US-A- 4 355 040

## Beschreibung

Die vorliegende Erfindung betrifft ein neues Verfahren zur Herstellung von gegebenenfalls 2-substituierten 5-Chlorimidazol-4-carbaldehyden der allgemeinen Formel 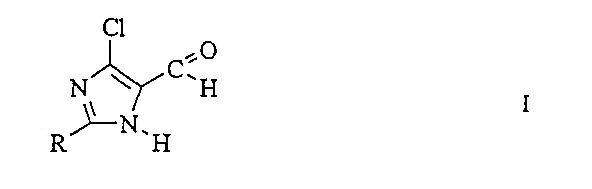 worin R Wasserstoff, eine C₁-C₆-Alkylgruppe, eine Alkenylgruppe mit bis zu 6 C-Atomen, eine Cyclopropyl-, Cyclobutyl-, Cyclopentyl- oder Cyclohexylgruppe oder eine gegebenenfalls mit Halogen, Alkyl-, Nitro- oder Aminogruppen substituierte Benzyl- oder Phenylgruppe bedeutet.

Die 2-substituierten 5-Chlorimidazol-4-carbaldehyde der allgemeinen Formel I sind wichtige Ausgangsprodukte zur Herstellung von blutdrucksenkenden Pharmazeutika (US-A-4 355 040) oder von herbizidwirksamen Verbindungen (DE-A-2804435).

Die US-A-4,345,935 beschreibt die Umsetzung eines 5-unsubstituierten 2,4-lmidazolidindions mit einem N,N-substituierten Formamiddimethylacetal zur Herstellung von 2,4-lmidazolidindionen mit einer Dialkylaminomethylengruppe in 5-Position des Imidazolidinsystems.

Zur Herstellung der erfindungsgemäßen 2-substituierten 5-Chlorimidazol-4-carbaldehyde der allgemeinen Formel I sind mehrere Wege bekannt.

Die EP-A-0 579 212 beschreibt ein Verfahren, bei dem ein 2-substituiertes 3,5-Dihydroimidazolin-4-on mit Phosphoroxychlorid in Gegenwart von N,N-Dimethylformamid umgesetzt wird. Das 3,5-Dihydroimidazolin-4-on wird durch Umsetzung eines substituierten Imidsäurealkylesters mit einem Glycinalkylester erhalten.

Die EP-A-0 614 892 beschreibt ein Verfahren, bei dem ein Glycinesterhydrohalogenid mit einem Imidsäureester zu einem 2-substituierten 3,5-Dihydroimidazol-4-on umgesetzt und dieses anschließend mit Phosphoroxychlorid zum Endprodukt chloriert wird.

Die EP-A-0 653 422 beschreibt ebenfalls die Umsetzung eines Glycinesterhydrohalogenids mit einem Imidsäureester, wobei das erhaltene 3,5-Dihydroimidazol-4-on anschließend zunächst mit einem N,N-substituierten Formamidacetal in das N,N-substituierte Aminomethylenimidazolinon überführt und dann chloriert wird.

Die US-A-4 355 040 beschreibt ein Verfahren, nach welchem 2-Amino-3,3-dichloracrylnitril mit einem Aldehyd zum entsprechenden Azomethinzwischenprodukt und weiter mit einem Halogenwasserstoff und Wasser zum 2-substituierten 5-Halogen-imidazol-4-carbaldehyd umgesetzt wird. Experimentelle Angaben fehlen in der genannten Patentschrift. Ein grosser Nachteil der Synthese besteht darin, dass das eingesetzte 2-Amino-3,3-dichloracrylnitril zunächst ausgehend von Dichloracetonitril, durch dessen Umsetzung mit Blausäure/Natriumcyanid hergestellt werden muss. Die äusserst toxischen Reaktionsteilnehmer und die damit verbundenen sicherheitstechnischen Massnahmen, die bereits für die Bereitstellung des Ausgangsproduktes notwendig sind, machen das Gesamtverfahren industriell untauglich.

Die US-A-4 355 040 offenbart in einer weiteren Variante ein 3-Stufen-Verfahren, worin in einer ersten Stufe ein Amidin-hydrochlorid mit Dihydroxyaceton mit hohem NH₃-Druck ringgeschlossen wird, der Imidazolalkohol halogeniert wird und schliesslich zum Aldehyd oxidiert wird.

Es hat sich gezeigt, dass für die Ringschlussreaktion Drucke von über 20 bar notwendig sind.

Die Oxidation des Alkohols funktioniert gemäss US-A-4 355 040 in Gegenwart von Chromoxid.

Es ist offensichtlich, dass eine Oxidation mit Schwermetalloxiden, die in der Regel nicht rezirkulierbar sind, nach heutigen ökologischen Gesichtspunkten nicht mehr verantwortbar ist.

Es bestand folglich die Aufgabe, ein Verfahren zu entwickeln, das die genannten Nachteile nicht aufweist.

Die Aufgabe konnte gelöst werden mit einem neuen Verfahren nach Anspruch 1.

Erfindungsgemäss wird ein gegebenenfalls 2-substituiertes 5-Chlorimidazol der allgemeinen Formel 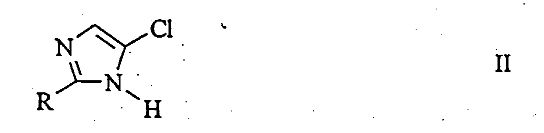 worin R die genannte Bedeutung hat, zunächst mit einem Amin der allgemeinen Formel 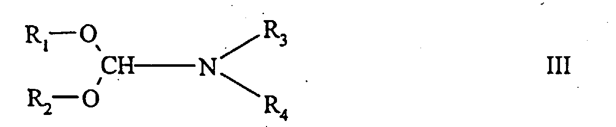 worin R₁ und R₂ gleich oder verschieden sind und eine C₁-C₆ -Alkylgruppe bedeuten oder R₁ und R₂ zusammen eine C₂-C₃ -Alkylenbrücke bilden, R₃ und R₄ gleich oder verschieden sind und eine C₁-C₆ -Alkylgruppe bedeuten oder R₃ und R₄ zusammen mit dem Amin-Stickstoff einen 5- oder 6-gliedrigen gesättigten Heterocyclus bilden, der gegebenenfalls Sauerstoff oder Stickstoff als zusätzliches Heteroatom enthält, zu einem gegebenenfalls 2-substituierten-5 -Chlorimidazol-4-ylidenmethylamin der allgemeinen Formel 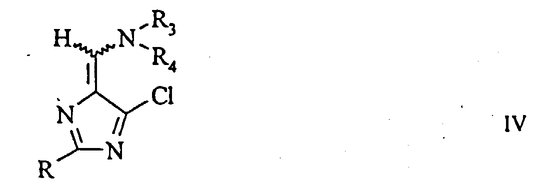 worin R, R₃ und R₄ die genannte Bedeutung haben, umgesetzt und danach zum Zielprodukt hydrolysiert.

R in der Bedeutung von Alkyl steht für geradkettiges oder verzweigtes C₁-C₆-Alkyl, wie z.B. Methyl-, Ethyl-, Propyl-, Isopropyl-, n-Butyl, sec. Butyl-, tert. Butyl-, Pentyl- und seine Isomeren, Hexyl- und seine Isomeren. Bevorzugte Alkylgruppe für R ist die n-Propylgruppe oder die n-Butylgruppe, R in der Bedeutung von Alkenyl- steht für geradkettiges oder verzweigtes Alkenyl mit bis zu 6 C-Atomen, wie z.B. für 1-Propenyl, 2-Propenyl, 1-Butenyl, 2-Butenyl, 3-Butenyl, Pentenyl und seine Isomeren oder Hexenyl und seine Isomeren. Bevorzugte Alkenylgruppe für R ist die 2- oder 3-Butenylgruppe.
Als Vertreter von Cycloalkylgruppen seien die Cyclopropyl-, die Cyclobutyl-, die Cyclopentyl- oder die Cyclohexylgruppe genannt.
Sowohl die Benzyl- als auch die Phenylgruppe kann Substituenten wie die genannten Alkylgruppen, Halogenatome, Nitrogruppen oder Aminogruppen enthalten.
Unter der Bezeichnung Halogen wird zweckmässig Fluor, Chlor, Brom oder Jod, vorzugsweise Chlor verstanden.

Das 2-substituierte 5-Chlorimidazol der allgemeinen Formel II als Ausgangsverbindung für das erfindungsgemässe Verfahren ist zugänglich durch Chlorierung des entsprechenden 3,5-Dihydroimidazol-4-ons gemäss EP-A 0 614 890.

Besonders geeignete Amine der allgemeinen Formel III sind solche, worin R₁ und R₂ gleichbedeutend sind und C₁-C₆-Alkyl wie z. B. Methyl, Ethyl, Propyl, Isopropyl, n-Butyl, sec.-Butyl, tert. Butyl, Pentyl und seine Isomeren oder Hexyl und seine Isomeren bedeutet oder worin R₁ und R₂ zusammen eine Ethylenbrücke bilden und worin R₃ und R₄ gleichbedeutend sind und für eine der genannten C₁-C₆-Alkylgruppen stehen.
R₃ und R₄ zusammen mit dem Amin-Stickstoff können auch einen 5- oder 6-gliedrigen gesättigten Heterocyclus bilden, der gegebenenfalls Sauerstoff oder Stickstoff als zusätzliches Heteroatom enthält. Folglich kann R₃ und R₄ zusammen mit dem Amin-Stickstoff einen Pyrrolidinring, einen Piperazinring, einen Piperidinring oder einen Morpholinring bilden. Bevorzugt steht R₁, R₂, R₃ und R₄ für Methyl oder Ethyl.
Besonders geeignete Amine der allgemeinen Formel III sind das Dimethoxymethyldimethylamin und das Diethoxymethyldiethylamin.

Die Amine der allgemeinen Formel III werden zweckmässig in einer Menge von 1 bis 5 Moläquivalenten, vorzugsweise von 1 bis 2 Moläquivalenten bezogen auf 1 Mol 2-subst. 5-Chlorimidazol der allgemeinen Formel II eingesetzt.

Eine selektive Umsetzung zum gegebenenfalls 2-substituierten 5-Chlorimidazol-4-ylidenmethylamin der allgemeinen Formel IV kann erreicht werden, wenn in Gegenwart eines tert. Amins als Base gearbeitet wird.

Zweckmässig wird das tert Amin in einer Menge von 0,05 bis 2,0 Moläquivalenten bezogen auf 1 Mol 2-subst. 5-Chlorimidazol der allgemeinen Formel II angewendet.
Bevorzugt wird ein Trialkylamin wie z. B. Triethylamin verwendet.

Die Umsetzung mit dem Amin der allgemeinen Formel III verläuft zweckmässig bei einer Temperatur zwischen 20 °C und 200 °C bevorzugt bei einer Temperatur von 100 °C bis 150 °C in Gegenwart eines inerten Lösungsmittels.
Tiefere Temperaturen sind zwar möglich, sie verlangsamen jedoch die Umsetzung und begünstigen Nebenreaktionen.

Als geeignete Lösungsmittel haben sich höhersiedende Aromaten wie z. B. Chlorbenzol oder Toluol bewährt.

Die resultierenden gegebenenfalls 2-substituierten 5-Chlorimidazol-4-ylidenmethylamine der allgemeinen Formel IV sind nicht literaturbekannt und bilden als zentrales Zwischenprodukt des erfindungsgemässen Verfahrens einen wesentlichen Bestandteil der Erfindung.

Besonders bevorzugte Verbindungen der allgemeinen Formel IV sind das (2-Butyl-5-chlorimidazol-4-ylidenmethyl)dimethylamin (R = n-Butyl, R₃, R₄ = Methyl), das (2-Butyl-5-chlorimidazol-4-ylidenmethyl)diethylamin (R = n-Butyl, R₃, R₄ = Ethyl), das (2-n-Propyl-5-chlorimidazol-4-ylidenmethyl)dimethylamin (R = n-Propyl, R₃, R₄ = Methyl) und das 2-n-Propyl-5-chlorimidazol-4-ylidenmethyl)diethylamin )diethylamin (R = n-Propyl, R₃, R₄ = Ethyl).

In der Regel werden die genannten Verbindungen der allgemeinen Formel IV im Verfahrensablauf nicht isoliert, sondern direkt der Hydrolyse zum Endprodukt unterworfen.

Die Hydrolysebedingungen sind nicht kritisch, d. h. die Hydrolyse kann in saurem, alkalischem oder neutralem Millieu erfolgen.

Die Isolierung des resultierenden 2-substituierten 5-Chlorimidazol-4-carbaldehyds kann auf fachmännisch übliche Weise, in der Regel durch Extraktion aus dem wässrigen Reaktionsgemisch, mit einem geeigneten Lösungsmittel erfolgen.

### Beispiele:

### Beispiel 1

### a) Herstellung von (2-n-Butyl-5-chlorimidazol-4-ylidenmethyl)dimethylamin

Eine Lösung von 2-n-Butyl-5-chlor-1H-imidazol (0,79 g, 5 mmol) und Dimethoxymethyldimethylamin (0,80 g, ca. 92%ig, 6,2 mmol) in Chlorbenzol (20 ml) wurde während 1,5 h bei 130 °C erhitzL Von der Reaktionslösung wurden 10 ml zur Trockene eingeengt und am Hochvakuum getrocknet Das so erhaltene dunkelbraune Öl (0,46 g) enthielt die Titelverbindung (ca. 80%ig nach H-NMR); dies entsprach einer Ausbeute von ca. 70% bezogen auf2-n-Butyl-5-chlor-1H-imidazol.
- ¹H-NMR (400 MHz, CDCl₃); δ: 0,92 (3H, t),
1,42 (2H, m),
1,74 (2H, m),
2,71 (2H, t);
3,36 (3H, s),
3,80 (3H, s),
7,37 (1H, s).
- MS;: 213 (M⁺), 171 (M - 42) (100%).

### b) Herstellung von (5-Chlor-2-propylimidazol-4-ylidenmethyl)dimethylamin

Eine Lösung von 5-Chlor-2-propyl-lH-imidazol (0,72 g, 5 mmol), Dimethoxymethyldimethylamin (0,77 g, ca. 92%ig, 6 mmol) und Triethylamin (0,10 g, 1 mmol) in Chlorbenzol (25 ml) würde während 1 h bei 60 °C und 2 h bei Rückfluss erhitzt. Entfernung des Lösungsmittels im Rotavapor lieferte die Titelverbindung in fast quantitativer Ausbeute.
- ¹H-NMR (400 MHz, CDCl₃); δ: 0,96 (3H, t);
1,78 (2H, m);
2,60 (2H, t);
3,39 (3H, s);
3,80 (3H, s);
7,38 (1H, s).
- MS;: 199 (M⁺), 171 (M - 28) (100%).

### Beispiel 2

### a) Herstellung von 2-n-Butyl-5-chlorimidazol-4-carbaldehyd (Lösungsmittel: Chlorbenzol)

Eine Lösung von 2-n-Butyl-5-chlor-1H-imidazol (1,59 g, 10 mmol), Dimethoxymethyldimethylamin (1,94 g, ca. 92%ig, 15 mmol) und Triethylamin (0,10 g, 1,0 mmol) in Chlorbenzol (44 ml) wurde 2,5 h bei 130 °C erhitzt, auf 30 °C gekühlt und zu 2 N HCl (50 ml) gegeben. Das Gemisch wurde 0,5 h bei Raumtemperatur gerührt und durch Zugabe von 30%iger Natronlauge (8,9 ml) von pH -0,34 auf pH 1,20 gestellt. Die Phasen wurden getrennt, und die wässrige Phase wurde zweimal mit je 30 ml Essigester extrahiert. Die vereinigten organischen Phasen wurden getrocknet (MgSO₄), filtriert, eingeengt und am Hochvakuum getrocknet Der so erhaltene Feststoff(1,67 g) enthielt die Titelverbindung (ca. 75%ig nach H-NMR); dies entsprach einer Ausbeute von ca. 67% bezogen auf 2-n-Butyl-5-chlor-1H-imidazol.
- ¹H-NMR (400 MHz, CDCl₃); δ: 0,93 (3H, t);
1,42 (2H, m);
1,75 (2H, m);
2,82 (2H, t);
9,61 (1H, s);
11,38(1H,br.s).

### b) Herstellung von 2-n-Butyl-5 2-n-Butyl-5-chlorimidazol-4-carbaldehyd (Lösungsmittel: Toluol)

Eine Lösung von 2-n-Butyl-5-chlor-1H-imidazol (1,59 g, 10 mmol), Dimethoxymethyldimethylamin (1,94 g, ca. 92%ig, 15 mmol) und Triethylamin (0,10 g, 1,0 mmol) in Toluol (35 ml) wurde-4,5 h bei 110 °C erhitzt, auf 30 °C gekühlt und zu 2 N HCl (50 ml) gegeben. Das Gemisch wurde 0,5 h bei Raumtemperatur gerührt und durch Zugabe von 30%iger Natronlauge (8,7 ml) von pH -0,28 auf pH 1,20 gestellt. Die Phasen wurden getrennt, und die wässrige Phase wurde zweimal mit je 30 ml Essigester extrahiert. Die vereinigten organischen Phasen wurden getrocknet (MgSO₄), filtriert, eingeengt und am Hochvakuum getrocknet. Der so erhaltene Feststoff (1,64 g) enthielt die Titelverbindung (ca. 80%ig nach H-NMR); dies entsprach einer Ausbeute von ca. 70% bezogen auf 2-n-Butyl-5-chlor-1H-imidazol.

### c) Herstellung von 2-Propyl-5-chlorimidazol-4-carbaldehyd

Eine Lösung von 5-Chlor-2-propyl-lH-imidazol (1,45 g, 10 mmol), Dimethoxymethyldimethylamin (1,55 g, ca. 92%ig, 12 mmol) und Triethylamin (0,10 g, 1 mmol) in Chlorbenzol (50 ml) wurde während 1 h bei 80 °C und 2,5 h bei Rückfluss erhitzt, auf Raumtemperatur gekühlt und auf 2 M Salzsäure (100 ml) gegossen. Der pH wurde durch Zugabe von 30%iger Natronlauge (19 ml) auf pH 1,97 gestellt, und das Gemisch wurde dreimal mit je 50 ml Essigester extrahiert. Die vereinigten organischen Phasen wurden getrocknet und am Rotavapor eingeengt. Ausbeute: 1,27 g (ca. 90%ig nach H-NMR), 66% bezogen auf 5-Chlor-2-propyl-1H-imidazol.
- ¹H-NMR (400 MHz, CDCl₃); δ: 1,01 (3H, t);
1,84 (2H, m);
2,83 (2H, t);
9,64 (1H, s);
11,56 (b. s, 1H).

## Patentansprüche

1. Verfahren zur Herstellung von gegebenenfalls 2-substituierten 5-Chlorimidazol-4-carbaldehyden der allgemeinen Formel 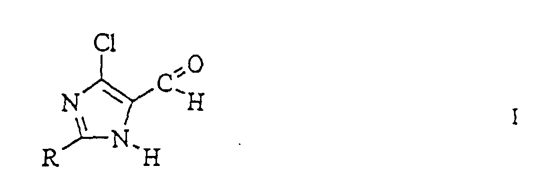 worin R Wasserstoff, eine C₁-C₆-Alkylgruppe, eine Alkenylgruppe mit bis zu 6 C-Atomen, eine Cyclopropyl-, Cyclobutyl-, Cyclopentyl- oder Cyclohexylgruppe oder eine gegebenenfalls mit Halogen, C₁-C₆-Alkyl-, Nitro- oder Aminogruppen substituierte Benzyl- oder Phenylgruppe bedeutet, dadurch gekennzeichnet, daß ein gegebenenfalls 2-substituiertes 5-Chlorimidazol der allgemeinen Formel 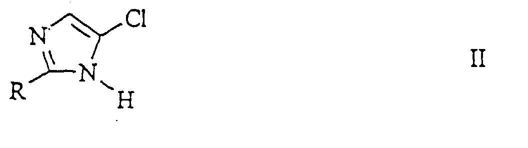 worin R die genannte Bedeutung hat, bei einer Temperatur zwischen 20°C und 200°C in Gegenwart eines inerten Lösungsmittels und eines tertiären Amins als Base mit einem Amin der allgemeinen Formel 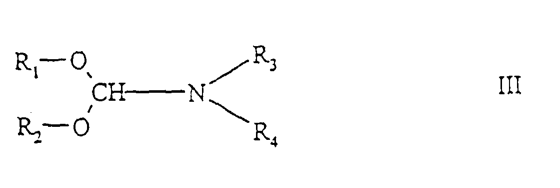 worin R₁ und R₂ gleich oder verschieden sind und eine C₁-C₆-Alkylgruppe bedeuten oder R₁ und R₂ zusammen eine C₂-C₃-Alkylenbrücke bilden, R₃ und R₄ gleich oder verschieden sind und eine C₁-C₆-Alkylgruppe bedeuten oder R₃ und R₄ zusammen mit dem Amin-Stickstoff einen 5- oder 6-giiedrigen gesättigten Heterocyclus bilden, der gegebenenfalls Sauerstoff oder Stickstoff als zusätzliches Heteroatom enthält, zu einem gegebenenfalls 2-substituierten 5-Chlorimidazol-4-ylidenmethylamin der allgemeinen Formel 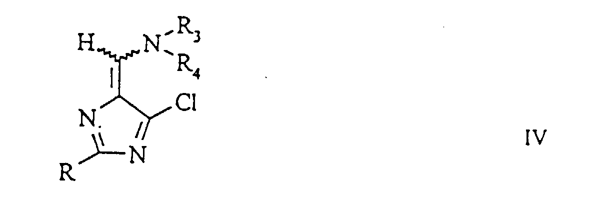 worin R, R₃ und R₄ die genannte Bedeutung haben, umgesetzt wird und schließlich zum Endprodukt hydrolysiert wird.

2. Verfahren nach Anspruch 1, dadurch gekennzeichnet, daß das 2-substituierte 5-Chlorimidazol-4-ylidenmethylamin der allgemeinen Formel IV nicht isoliert wird.

3. Gegebenenfalls 2-substituiertes 5-Chlorimidazol-4-ylidenmethylamin der allgemeinen Formel 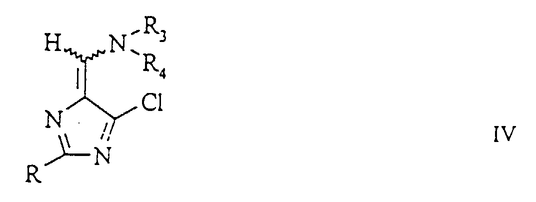 worin R, R₃ und R₄ die in Anspruch 1 genannte Bedeutung haben.

4. (2-n-Butyl-5-chloriniidazol-4-ylidenmethyl)dimethylamin

5. (2-n-Butyl-5-chlorimidazol-4-ylidenmethyi)diethylamin

6. (2-n-Propyl-5-chlorimidazol-4-ylidenmethyl)dimethylamin

7. (2-n-Propyl-5-chlorimidazol-4-ylidenmethyl)diethylamin

## Claims

1. Process for the preparation of optionally 2-substituted 5-chloroimidazole-4-carbaldehydes corresponding to the general formula 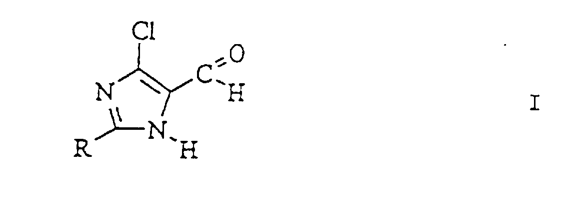 wherein R denotes hydrogen, a C₁-C₆-alkyl group, an alkenyl group having up to 6 C atoms, a cyclopropyl, cyclobutyl, cyclopentyl or cyclohexyl group, or a benzyl or phenyl group optionally substituted with halogen, C₁-C₆-alkyl, nitro or amino groups, characterised in that an optionally 2-substituted 5-chloroimidazole corresponding to the general formula 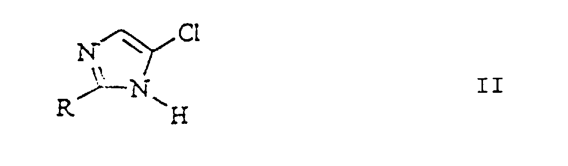 wherein R has the meaning given above, is reacted at a temperature of between 20°C and 200°C, in the presence of an inert solvent and of a tertiary amine as base, with an amine corresponding to the general formula 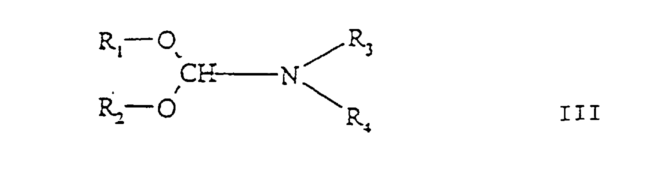 wherein R₁ and R₂ are identical or different and denote a C₁-C₆-alkyl group or R₁ and R₂ together form a C₂-C₃-alkylene bridge, R₃ and R₄ are identical or different and denote a C₁-C₆-alkyl group or R₃ and R₄ together with the amine nitrogen atom form a 5- or 6-membered saturated heterocycle which optionally contains oxygen or nitrogen as additional hetero atom, to produce an optionally 2-substituted 5-chloroimidazol-4-ylidenemethylamine corresponding to the general formula 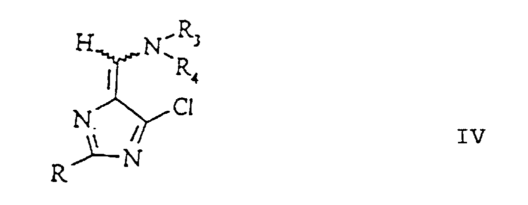 wherein R, R₃ and R₄ have the meanings given above, and is finally hydrolysed to the end product.

2. Process according to claim 1, characterised in that the 2-substituted 5-chloroimidazol-4-ylidenemethylamine corresponding to the general formula IV is not isolated.

3. Optionally 2-substituted 5-chloroimidazol-4-ylidenemethylamine corresponding to the general formula IV 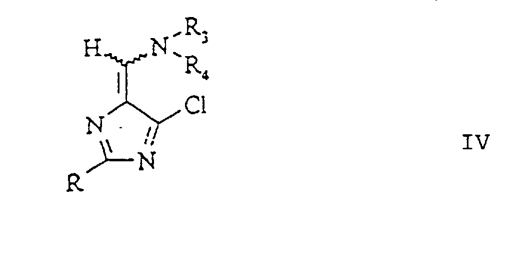 wherein R, R₃ and R₄ have the meanings given in claim 1.

4. (2-n-butyl-5-chloroimidazol-4-ylidenemethyl)dimethylamine

5. (2-n-butyl-5-chloroimidazol-4-ylidenemethyl)diethylamine

6. (2-n-propyl-5-chloroimidazol-4-ylidenemethyl)dimethylamine

7. (2-n-propyl-5-chloroimidazol-4-ylidenemethyl)diethylamine

## Revendications

1. Procédé de préparation de 5-chloroimidazole-4-carbaldéhydes éventuellement substitués en position 2 de formule générale 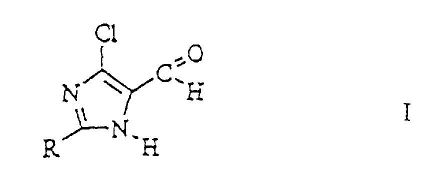 dans laquelle R représente un atome d'hydrogène, un groupe alkyle en C₁-C₆, un groupe alcényle ayant jusqu'à 6 atomes de carbone, un groupe cyclopropyle, cyclobutyle, cyclopentyle ou cyclohexyle ou un groupe benzyle ou phényle éventuellement substitué par des groupes halogéno, alkyle en C₁-C₆, nitro ou amino, caractérisé en ce que l'on fait réagir un 5-chloroimidazole éventuellement substitué en position 2 de formule générale 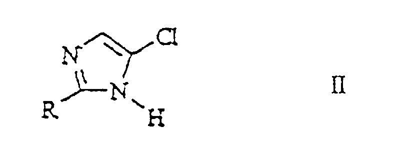 dans laquelle R a la signification indiquée, à une température comprise entre 20°C et 200°C, en présence d'un solvant inerte et d'une amine tertiaire comme base, avec une amine de formule générale 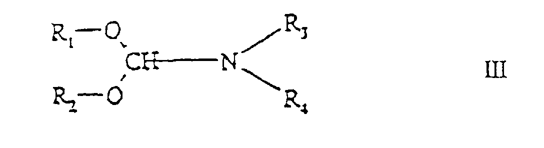 dans laquelle R₁ et R₂ sont identiques ou différents et représentent un groupe alkyle en C₁-C₆ ou R₁ et R₂ forment ensemble un pont alkylène en C₂-C₃, R₃ et R₄ sont identiques ou différents et représentent un groupe alkyle en C₁-C₆ ou R₃ et R₄ forment ensemble, avec l'atome d'azote, un hétérocycle saturé de 5 ou 6 chaînons contenant éventuellement de l'oxygène ou de l'azote comme hétéroatome supplémentaire, pour former une 5-chloroimidazol-4-ylidèneméthylamine éventuellement substituée en position 2 de formule générale 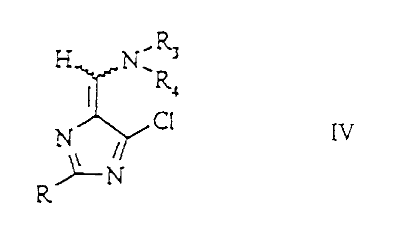 dans laquelle R, R₃ et R₄ ont la signification indiquée, et on effectue finalement une hydrolyse pour obtenir le produit final.

2. Procédé selon la revendication 1, caractérisé en ce que l'on n'isole pas la 5-chloroimidazol-4-ylidèneméthylamine éventuellement substituée en position 2 de formule générale IV.

3. 5-chloroimidazol-4-ylidèneméthylamine éventuellement substituée en position 2 de formule générale 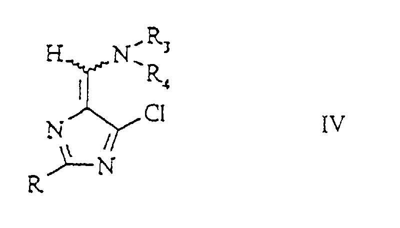 dans laquelle R, R₃ et R₄ ont la signification indiquée dans la revendication 1.

4. (2-n-butyl-5-chloroimidazol-4-ylidèneméthyl)diméthylamine.

5. (2-n-butyl-5-chloroimidazol-4-ylidèneméthyl)diéthylamine.

6. (2-n-propyl-5-chloroimidazol-4-ylidèneméthyl)diméthylamine.

7. (2-n-propyl-5-chloroimidazol-4-ylidèneméthyl)diéthylamine.
